# EUROPEAN PATENT SPECIFICATION

(11) **EP 3 677 189 B1**
(45) Date of publication and mention of the grant of the patent: **09.04.2025**
(21) Application number: 20150022.0
(22) Date of filing: 02.01.2020
(51) Int. Cl.: A61B 8/00, B32B 7/12, B32B 27/08, B32B 27/40

(54) **ADHESIVE HYDROPHILIC PAD FOR ULTRASOUND TRANSDUCER**
HAFTENDES HYDROPHILES KISSEN FÜR ULTRASCHALLWANDLER
TAMPON HYDROPHILE ADHÉSIF POUR TRANSDUCTEUR ULTRASONORE

(30) Priority: 03.01.2019 US 201916238729
(43) Date of publication of application: 08.07.2020
(73) Proprietor: Civco Medical Instruments Co., Inc., Kalona, IA 52247 (US)
(72) Inventor: Wagner, Geoffrey Scott, Fairfax, Iowa 52228 (US); Swartz, Alexas Marin, Coralville, Iowa 52241 (US); Swartz, Ryan Albert, Coralville, Iowa 52241 (US); Nordgren, Gregory Nephi, North Liberty, Iowa 52317 (US)
(74) Representative: Zacco Denmark A/S

(56) References cited:
- US-A1- 2007 016 053
- US-A1- 2015 305 709
- US-A1- 2018 310 912

## Description

### BACKGROUND

This disclosure relates to medical devices and more particularly to ultrasound transducers and devices for covering the ultrasound transducer for use in external, intraoperative, or endocavity applications.

Ultrasound transducers are commonly used in clean, but non-sterile environments, such as patient examination rooms. In many typical ultrasound procedures, such as prenatal abdominal ultrasounds, bladder or other organ screenings, transesophageal echocardiography, etc., an acoustic ultrasound gel is applied to a supine patient's abdomen and an ultrasound transducer is positioned to contact the gel and is moved around the abdomen to acquire ultrasound images. Once the procedure is complete, both the patient and the ultrasound transducer must be cleaned of gel. In circumstances in which time between procedures is a concern, the cleaning process negatively impacts productivity. Examples of known removable interfaces for covering ultrasound transducers are disclosed in Documents US2018310912A1 , US2007016053A1 , US2015305709A1.

### BRIEF DESCRIPTION OF THE DRAWINGS

Fig. 1A-1C illustrate an environment in which embodiments described herein may be implemented;
Figs. 2A-2D are cross-sectional views of an exemplary implementation of the interface pad of Fig. 1; and
Figs. 3A-3D are flow charts illustrating exemplary processes of forming and using an ultrasound transducer interface pad in accordance with embodiments described herein.

### DETAILED DESCRIPTION OF THE PREFERRED EMBODIMENTS

The following detailed description refers to the accompanying drawings. The same reference numbers in different drawings may identify the same or similar elements.

The invention is set out in the appended set of claims. The present invention provides an ultrasound transducer interface pad, comprising: a first substrate layer (120) having a first surface and a second surface; a hydrophilic layer (130) formed on the first surface of the first substrate layer, wherein the hydrophilic layer is configured to be hydrated to provide an acoustic coupling between an ultrasound transducer and a patient; a second substrate layer (140) having a third surface and a fourth surface; a first adhesive layer (125) formed on the third surface of the second substrate layer and configured to adhere to the second surface of the first substrate layer; and a second adhesive layer (145) formed on the fourth surface of the second substrate layer and configured to adhere to an operational portion of an ultrasound transducer characterized in that: the first adhesive layer comprises a non-silicon-based adhesive including an acrylic rubber-based adhesive material or a synthetic rubber-based adhesive material and the second adhesive layer comprises a silicone-based adhesive, and wherein the non-silicone-based adhesive exhibits a first removal force greater than a second removal force of the silicone-based adhesive.

In a further aspect, the present invention provides a method of making a disposable ultrasound transducer interface pad, the method comprising: applying a hydrophilic material to a first surface of a first substrate layer (120) to form a hydrophilic layer (130), wherein the hydrophilic material is configured to be hydrated to provide an acoustic coupling between the patient and an ultrasound transducer; curing the hydrophilic layer; applying a first adhesive to a third surface of a second substrate layer (140) to form a first adhesive layer (125), applying a second adhesive to a fourth surface of the second substrate layer to form a second adhesive layer (145); bonding the first substrate layer to the second substrate layer via the first adhesive layer; and cutting the bonded first substrate layer and second substrate layer to a size and a shape to form the disposable ultrasound transducer interface pad, wherein the first adhesive layer comprises a non-silicone-based adhesive and the second adhesive layer comprises a silicone-based adhesive, and wherein the non-silicone-based adhesive exhibits a first removal force greater than a second removal force of the silicone-based adhesive.

Implementations described herein relate to materials for providing an effective and easy to use interface between an ultrasound transducer and a patient. Consistent with one implementation described herein, a disposable transducer interface includes a multilayer configuration, hereinafter referred to as a "pad," for engaging an operating end of the transducer on one side and a patient's skin on the opposite side. The disclosed multilayer interface pad includes a carrier layer, with an adhesive layer and a hydrophilic layer applied to opposing sides of the carrier layer. During use, the adhesive layer side of the pad removably adheres to the transducer (or patient) to provide a positive, consistent coupling between the pad and the transducer. The hydrophilic layer is then hydrated to provide a positive acoustic coupling that facilitates clear and efficient transmission of ultrasound signals therethrough and eliminates the need to use traditional acoustic coupling gel.

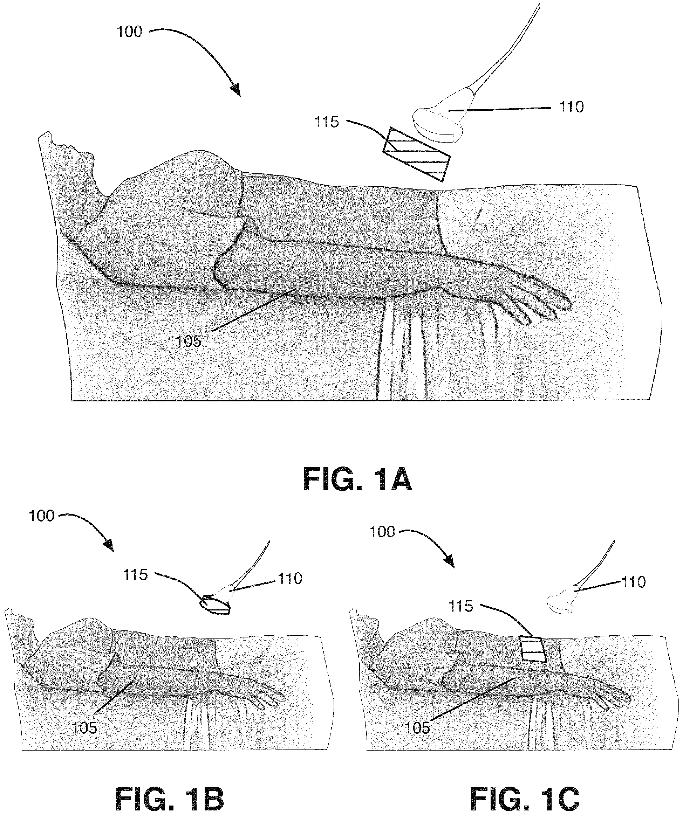

Fig. 1A-1C illustrate an environment 100 in which embodiments described herein may be implemented. As shown in Fig. 1A, environment 100 includes a patient 105, an ultrasound transducer 110, and an interface pad 115. During use, as shown in Figs. 1B and 1C, interface pad 115 may be used in one of two manners. In a first embodiment, as shown in Fig. 1B, interface pad 115 may be adhered, as described below, to the operational end of ultrasound transducer 110, while in the second embodiment, as shown in Fig. 1C, interface pad 115 may be adhered to patient 105. In either embodiment, once affixed to either transducer 110 or patient 105, pad 115 may be hydrated by applying a liquid, such as water, saline, lidocaine, chloraprep, isopropyl alcohol, or other like solution to the exposed surface to form an acoustically efficient interface and allow for easy movement (i.e., sliding) between transducer 110 and interface pad 115. In some embodiments, a patient's bodily fluid or excretions may be sufficient to hydrate pad 115. In such embodiments, external or added hydrating solutions may not be necessary.

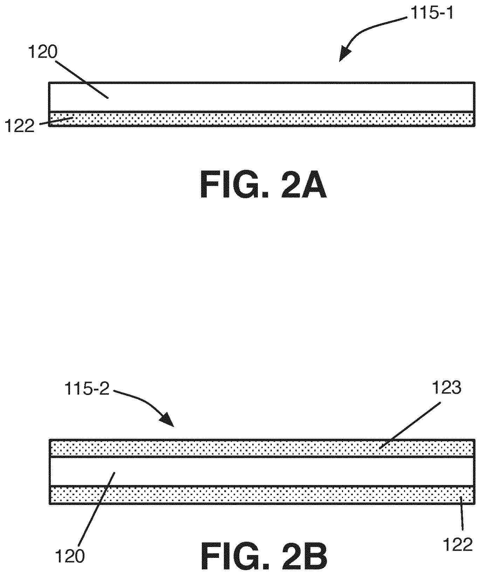

Fig. 2A-2D illustrate cross-sectional views of exemplary implementations of interface pad 115, depicted as interface pads 115-1 to 115-4, respectively. As shown in Fig. 2A, interface pad 115-1 includes a substrate layer 120, such as a polyurethane carrier or material having a thickness ranging from approximately 0.025 to 1.0 mm. Consistent with implementations described herein, substrate layer 120 may be formed in either a planar or non-planar (e.g., shaped) configuration depending on application. For example, in some embodiments, substrate layer 120 may have a shaped (e.g., three-dimensional) configuration corresponding to the ultrasound transducer onto which it is to be applied. In other embodiments, substrate layer 120 may be formed as a planar layer usable with a number of different transducers and in a variety of procedures. Furthermore, consistent with embodiments described here, substrate layer 120 may be formed in any feasible manner to accomplish the desired dimensions. For example, substrate layer 120 may be formed by extrusion, dipping, molding, chemical deposition, etc.

Consistent with embodiments described herein, interface pad 115-1 further includes a hydrophilic coating layer 122 applied to one side of substrate layer 120. In this configuration, hydrophilic coating layer 122 is provided on an outside of interface pad 115 relative to transducer 110.

In one embodiment, hydrophilic coating layer 122 includes an ultra-violet (UV) light or heat curable materials, such as polyvinylpyrrolidone/polyurethane (PVP/PU) or poly methacrylate (PM), having a thickness in the range of approximately 2 to 5 microns. During manufacture, hydrophilic coating layer 122 may be applied to the substrate layer 120 and cured via exposure to UV light or exposing the layer to heat.

During use, an acoustic coupling gel may be applied to an inside of substrate layer 120 prior to applying interface pad 115-1 to the ultrasound probe. Next, hydrophilic coating layer 122 may be activated using only water or saline to provide the requisite acoustic coupling interface between transducer 110 and patient 105.

Consistent with embodiments described herein, interface pad 115-1 may be formed of any suitable shape or dimensions consistent with the particular ultrasound transducer or patient body part with which it is to be used. For example, in one embodiment, interface pad 115-1 may be formed in a rectangular configuration having a length of approximately 5 inches and a width of approximately 3.25 inches.

Fig. 2B illustrates an embodiment of interface pad 115-2 that includes a second hydrophilic coating layer 123 applied on a side of substrate layer 120 opposite from hydrophilic coating layer 122. During use, hydrophilic coating layers 122 and 123 may each be activated using only water or saline to provide the requisite acoustic coupling interface between transducer 110 and patient 105.

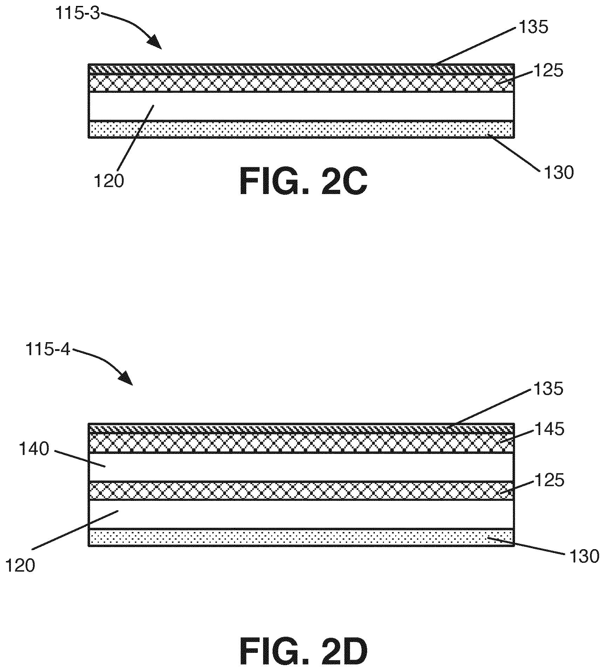

In another implementation, as shown in Fig. 2C, interface pad 115-3 includes substrate layer 120, an adhesive layer 125, a hydrophilic coating layer 130, and a removable release layer 135. In one embodiment, substrate layer 120 comprises a polyurethane film carrier or material, such as polyether polyurethane having a thickness ranging from approximately 0.025 to 1.00 millimeters (mm).

In other embodiments, adhesive layer 125 may include an acrylic or synthetic rubber-based adhesive material. Such non-silicone-based adhesives, may exhibit significantly higher removal forces (e.g., as high as 16.7N per 25 mm). An adhesive having a higher removal force may be desirable in some circumstances, such as where slippage of the pad during use is a concern.

Consistent with embodiments described herein, adhesive layer 125 is applied (e.g., coated) onto substrate layer 120 at a coat weight ranging from approximately 100 to 200 grams per square meter (gsm), and preferably at a coat weight of 150 gsm, resulting in adhesive layer 125 having an applied thickness ranging from 0.025 to 0.2 mm (e.g., 0.15 mm).

As shown in Fig. 2C, hydrophilic coating layer 130 is applied to substrate layer 120 on an opposite side of substrate layer 120 relative to adhesive layer 125. During manufacture and prior to use, interface pad 115-3 includes a release layer 135 (also referred to as a liner or release liner) that is provided on adhesive layer 125 to protect the tackiness of adhesive layer 125 and to prevent adhesive layer 125 from adhering to other items or itself prior to use. In one implementation, release layer 135 comprises a polycarbonate layer. Consistent with embodiments described herein, release layer 135 is removed (e.g., peeled off) prior to using interface pad 115-3, e.g., prior to adhering interface pad 115 to transducer 110/patient 105. In some embodiments, release layer 135 may include an edge area or slit that allows release layer 135 to be easily removed when interface pad 115-3 is ready for use.

Although interface pad 115-3 of Fig. 2C is described above as including three distinct layers 120-130 and a release layer 135, in other implementations, interface pad 115 may be formed of only two layers, with an adhesive layer 125 being applied directly to hydrophilic coating layer 130, without the requirement of an underlying polyurethane substrate layer.

Fig. 2D depicts interface pad 115-4 that includes substrate layer 120 and hydrophilic layer 130, as described above in relation to Fig. 2C. In addition, interface pad 115-4 includes a second substrate layer 140 sandwiched between adhesive layer 125 and a second adhesive layer 145. In some implementations, this combination of adhesive layer 125, second substrate layer 140, and second adhesive layer 145 is formed independently of substrate layer 120 and hydrophilic coating layer 130. During manufacture, adhesive layer 125 (previously joined with second substrate 140 and second adhesive layer 145) is joined with initial substrate 120, the opposite side of which includes hydrophilic coating layer 130.

In one embodiment, second substrate layer 140 and initial substrate layer 120 each comprises a polyurethane film carrier or material, such as polyether polyurethane having a thickness ranging from approximately 0.025 to 1.00 millimeters (mm). In some implementations, adhesive layers 125/145 may include a silicone-based adhesive, having, for example, an adhesion (or removal force) of between 0.2 and 0.8 Newtons (N) per 25 millimeters (mm). The relatively low removal force of such a silicon-based adhesive renders interface pad 115-4 generally repositionable after initial deployment. Furthermore, such silicone-based adhesives are capable of sticking to itself without destroying the product during initial deployment, repositioning or removing.

Adhesive layer 145 include an acrylic or synthetic rubber-based adhesive material. The non-silicone-based adhesives exhibit significantly higher removal forces (e.g., as high as 16.7N per 25 mm). An adhesive having a higher removal force may be desirable in some circumstances, such as where slippage of the pad during use is a concern.

Thus, adhesive layer 125 include a different material than adhesive layer 145, i.e. adhesive layer 125 include an acrylic or synthetic rubber-based adhesive material and adhesive layer 145 include silicone-based adhesive.

Consistent with embodiments described herein, adhesive layers 125/145 are applied (e.g., coated) onto second substrate layer 140 at a coat weight ranging from approximately 100 to 200 grams per square meter (gsm), and preferably at a coat weight of 150 gsm, resulting in adhesive layers 125/145 each having an applied thickness ranging from 0.025 to 0.2 mm (e.g., 0.15 mm).

During manufacture and prior to use, interface pad 115-4 also includes release layer 135 (also referred to as a liner or release liner) that is provided on second adhesive layer 145 to protect the tackiness of second adhesive layer 145 and to prevent second adhesive layer 145 from adhering to other items or itself prior to use. In one implementation, release layer 135 comprises a polycarbonate layer. Consistent with embodiments described herein, release layer 135 is removed (e.g., peeled off) prior to using interface pad 115, e.g., prior to adhering interface pad 115-4 to transducer 110/patient 105. In some embodiments, release layer 135 may include an edge area or slit that allows release layer 135 to be easily removed when interface pad 115 is ready for use.

In other implementations, although not depicted in Fig. 2D, a second release layer may be applied to a surface of adhesive layer 125 opposite to second substrate 140 prior to application of adhesive layer 125 to initial substrate 120. In this manner, the adhesive layer 125/second substrate 140/second adhesive layer 145 combination may be protectively stored prior to use.

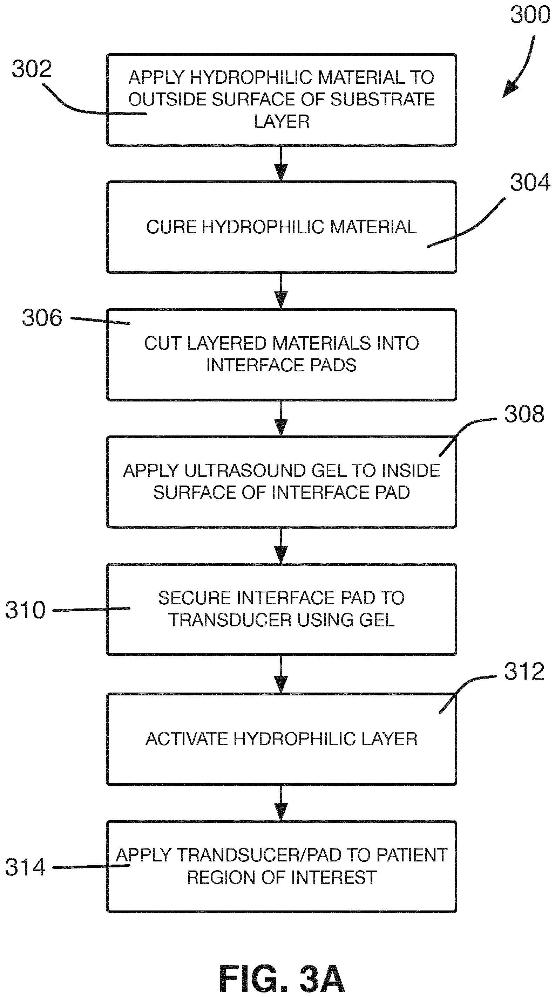

Figs. 3A-3D illustrate flow charts illustrating exemplary processes 300, 325, and 350, and 375, respectively of forming and using an ultrasound transducer interface pad in accordance with embodiments described herein, with process 300 corresponding to the embodiment of Fig. 2A, process 325 corresponding to the embodiment of Fig. 2B, process 350 corresponding to the embodiment of Fig. 2C, and process 375 corresponding to the embodiment of Fig. 2D.

Referring to Fig. 3A, a hydrophilic material may be coated onto one side of a substrate material (block 302). For example, hydrophilic layer 122 may be coated (e.g., sprayed, brushed, etc.) on a side of a sheet of polyurethane substrate. As described above, as a precursor to the application of hydrophilic layer 122, the substrate material (e.g., substrate layer 120) may be initially formed into a desired configuration using a suitable manufacturing technique, such as extrusion, dipping, molding, deposition, etc.

At block 304, the hydrophilic material is cured, such as via heat or UV light. At block 306, one or more interface pads 115 are cut to a desired size and/or shape, such as with a die cut machine.

At block 308, a traditional ultrasound coupling gel is applied to an inside surface of an interface pad 115-1. Next, interface pad 115-1 is applied to an operating end of ultrasound transducer 110 to secure interface pad 115-1 to transducer 110 (block 310). Next, the hydrophilic layer is activated (block 312). For example, a water or saline may be applied to hydrophilic layer 122. Finally, the ultrasound transducer with the activated interface pad secured thereto is applied to a region of interest on a patient (block 314).

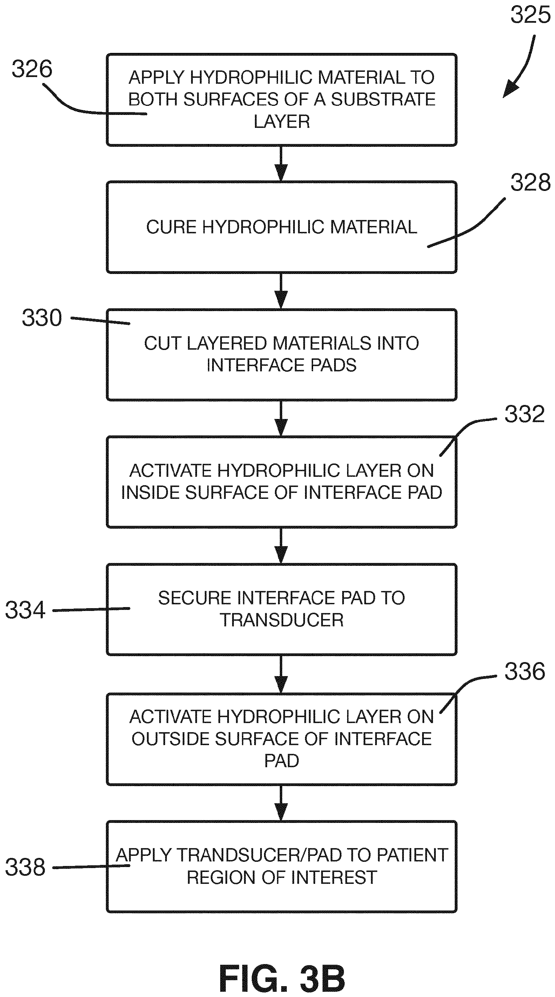

Referring to Fig. 3B, a hydrophilic material may be coated onto both sides of a substrate material (block 326). For example, hydrophilic layers 122 and 123 may be coated (e.g., sprayed, brushed, etc.) onto the sides of a sheet of polyurethane substrate. At block 328, the hydrophilic material is cured, such as via heat or UV light. In some implementations, hydrophilic layer application and curing are done independently for each of layers 122 and 123. At block 330, one or more interface pads 115-2 are cut to a desired size and/or shape, such as with a die cut machine.

At block 332, one of the hydrophilic layers is activated. For example, hydrophilic layer 123 is activated using water or saline. Next, interface pad 115-2 is applied to an operating end of ultrasound transducer 110 to secure interface pad 115-2 to transducer 110 via the activated hydrophilic layer 123 (block 334). Next, the other hydrophilic layer is activated (block 336). For example, a water or saline may be applied to hydrophilic layer 122. Finally, the ultrasound transducer with the activated interface pad secured thereto is applied to a region of interest on a patient (block 338).

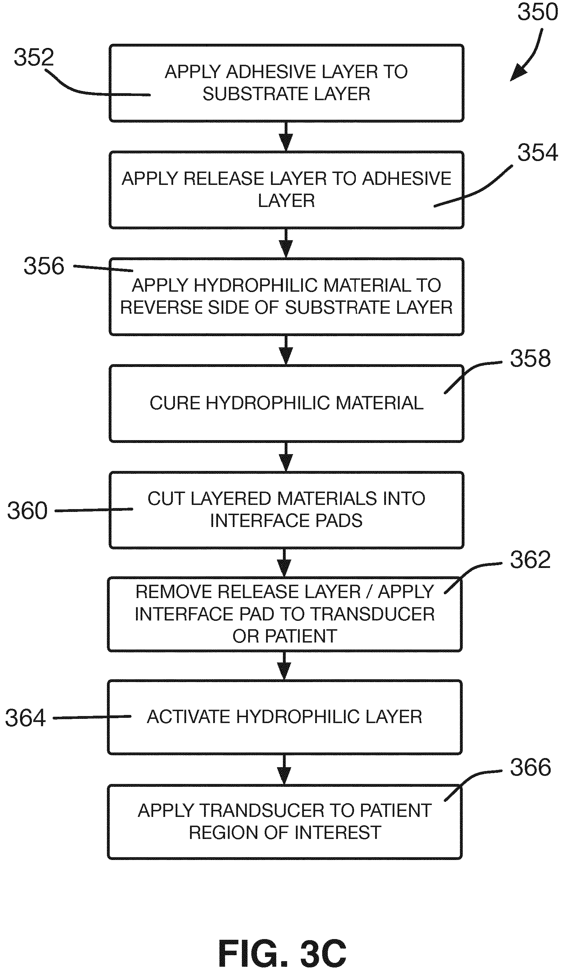

Referring to Fig. 3C, an adhesive layer is initially applied to a substrate layer (block 352). For example, a silicone adhesive material may be coated (e.g., poured, sprayed, brushed, etc.) onto a first surface of a polyurethane substrate layer, such as substrate layer 120 to form the adhesive layer. Next, a release layer, such as a polymeric or paper layer, may be applied to the adhesive layer to prevent the adhesive from losing tackiness or sticking to unintended materials (block 354). For example, release layer 135 may be applied to a tacky side of adhesive layer 125.

Next, a hydrophilic material may be coated on a reverse side of the substrate layer (block 356). For example, hydrophilic layer 130 may be coated (e.g., poured, sprayed, brushed, etc.) on a side of substrate 120 opposite to adhesive layer 125. At block 358, the hydrophilic material is cured, such as via heat or UV light. At block 360, one or more interface pads 115-3 are cut to a desired size and/or shape from the layered materials, such as with a die cut machine.

At block 362, the release layer is removed, and the adhesive layer is applied to either to an operating end of an ultrasound transducer or directly to the region of interest on the patient. For example, release layer 135 is removed to expose the tacky side of adhesive layer 125 and the adhesive layer 125 is then applied to transducer 110 or patient 105. Next, at block 364, the hydrophilic layer is activated. For example, hydrophilic layer 130 is activated using water or saline. Finally, either the ultrasound transducer with the activated interface pad 115-3 secured thereto is applied to a region of interest on a patient or the ultrasound transducer is applied to the activated interface pad 115-3 secured to the patient (block 366).

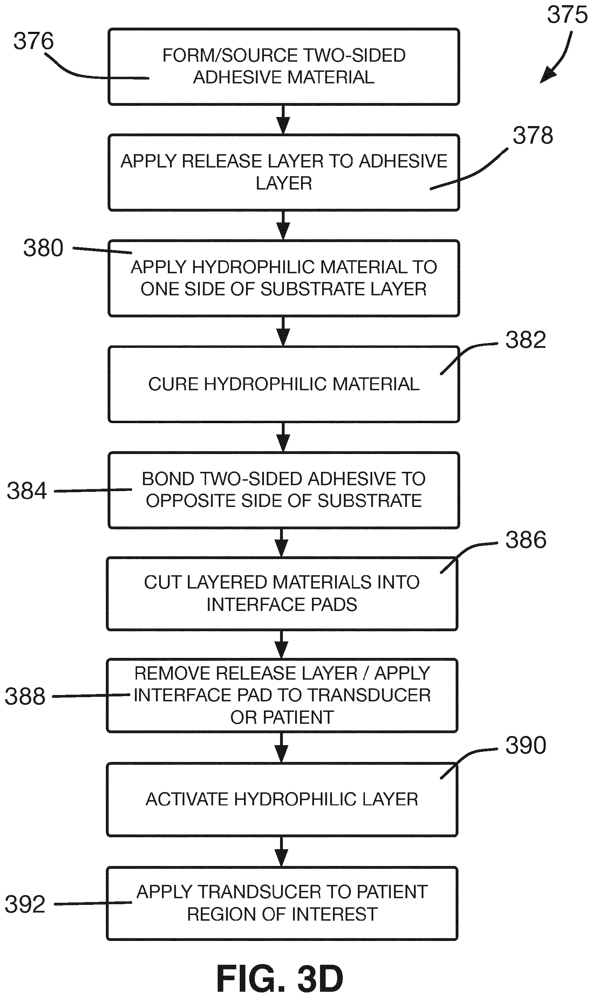

Referring to Fig. 3D, a two-sided adhesive is formed and/or sourced (block 376). For example, a material having second substrate 140, sandwiched between adhesive layer 125 and second adhesive layer 145 is formed. One or more release layers, such as a polymeric or paper layer, may be applied to the adhesive layers 125/145 to prevent the adhesive from losing tackiness or sticking to unintended materials (block 378).

Next, a hydrophilic material may be coated on a reverse side of the substrate layer (block 380). For example, hydrophilic layer 130 may be coated (e.g., poured, sprayed, brushed, etc.) one side of substrate 120. At block 382, the hydrophilic material is cured, such as via heat or UV light. At block 384, the two-sided adhesive of blocks 376/378 is bonded to the side of the substrate layer opposite the hydrophilic layer. Next, one or more interface pads 115-4 are cut to a desired size and/or shape from the layered materials, such as with a die cut machine (block 386).

At block 388, the release layer is removed, and the adhesive layer is applied to either to an operating end of an ultrasound transducer or directly to the region of interest on the patient. For example, release layer 135 is removed to expose the tacky side of adhesive layer 145 and adhesive layer 155 is then applied to transducer 110 or patient 105. Next, at block 390, the hydrophilic layer is activated. For example, hydrophilic layer 130 is activated using water or saline. Finally, either the ultrasound transducer with the activated interface pad 115-4 secured thereto is applied to a region of interest on a patient or the ultrasound transducer is applied to the activated interface pad 115-4 secured to the patient (block 392).

Consistent with embodiments described herein, the interface pad may be packaged as either a sterile or a non-sterile product for use in different medical environments or circumstances.

.

No element, act, or instruction used in the description of the present application should be construed as critical or essential to the invention ,which scope is defined by the appended claims, unless explicitly described as such. Also, as used herein, the article "a" is intended to include one or more items. Further, the phrase "based on" is intended to mean "based, at least in part, on" unless explicitly stated otherwise.

Use of ordinal terms such as "first," "second," "third," etc., in the claims to modify a claim element does not by itself connote any priority, precedence, or order of one claim element over another, the temporal order in which acts of a method are performed, the temporal order in which instructions executed by a device are performed, etc., but are used merely as labels to distinguish one claim element having a certain name from another element having a same name (but for use of the ordinal term) to distinguish the claim elements.

## Claims

1. An ultrasound transducer interface pad, comprising:
a first substrate layer (120) having a first surface and a second surface;
a hydrophilic layer (130) formed on the first surface of the first substrate layer, wherein the hydrophilic layer is configured to be hydrated to provide an acoustic coupling between an ultrasound transducer and a patient;
a second substrate layer (140) having a third surface and a fourth surface;
a first adhesive layer (125) formed on the third surface of the second substrate layer and configured to adhere to the second surface of the first substrate layer; and
a second adhesive layer (145) formed on the fourth surface of the second substrate layer and configured to adhere to an operational portion of an ultrasound transducer,
**characterized in that**:
the first adhesive layer comprises a non-silicon-based adhesive including an acrylic rubber-based adhesive material or a synthetic rubber-based adhesive material,
and the second adhesive layer comprises a silicone-based adhesive, and wherein the non-silicone-based adhesive exhibits a first removal force greater than a second removal force of the silicone-based adhesive.

2. The ultrasound transducer interface pad of claim 1, wherein at least one of the first substrate layer and the second substrate layer comprises a polyurethane film material.

3. The ultrasound transducer interface pad of claim 2, wherein the polyurethane film material has a thickness ranging from approximately 0.025 millimeter (mm) to 1.00 mm.

4. The ultrasound transducer interface pad of any of the preceding claims, wherein the hydrophilic layer comprises one of: an ultra-violet (UV) light curable hydrophilic material or a heat curable hydrophilic material.

5. The ultrasound transducer interface pad of claim 1, wherein the second adhesive layer has a thickness ranging from approximately 0.025 millimeter (mm) to 0.2 mm.

6. The ultrasound transducer interface pad of claim 5, wherein the second adhesive layer has a coat weight in a range of 100 grams per square meter (gsm) to 200 gsm.

7. The ultrasound transducer interface pad of any of the preceding claims, further comprising:
a release layer provided over the second adhesive layer,
wherein the release layer is to be removed prior to adhering the second adhesive layer to the ultrasound transducer.

8. A method of making a disposable ultrasound transducer interface pad, the method comprising:
applying a hydrophilic material to a first surface of a first substrate layer (120) to form a hydrophilic layer (130),
wherein the hydrophilic material is configured to be hydrated to provide an acoustic coupling between the patient and an ultrasound transducer;
curing the hydrophilic layer;
applying a first adhesive to a third surface of a second substrate layer (140) to form a first adhesive layer (125),
applying a second adhesive to a fourth surface of the second substrate layer to form a second adhesive layer (145);
bonding the first substrate layer to the second substrate layer via the first adhesive layer; and
cutting the bonded first substrate layer and second substrate layer to a size and a shape to form the disposable ultrasound transducer interface pad,
wherein the first adhesive layer comprises a non-silicone-based adhesive and the second adhesive layer comprises a silicone-based adhesive, and wherein the non-silicone-based adhesive exhibits a first removal force greater than a second removal force of the silicone-based adhesive.

9. The method of claim 8, wherein at least one of the first substrate layer or the second substrate layer comprises a polyurethane material.

10. The method of claim 9, wherein the polyurethane material has a thickness ranging from approximately 0.025 millimeter (mm) to 1.00-mm.

11. The method of any of claims 8-10, further comprising:
applying a release layer to the second adhesive layer.

12. The method of claim 8, wherein the second adhesive layer has a thickness ranging from approximately 0.025 millimeter (mm) to 0.2 mm.

13. The method of claim 12, wherein applying a second adhesive comprises:
coating the silicone-based adhesive at a coat weight in a range of 100 grams per square meter (gsm) to 200 gsm.

14. The method of any of claims 8-13, wherein the hydrophilic material comprises one of: an ultraviolet (UV) light curable hydrophilic material or a heat curable hydrophilic material, and wherein curing the hydrophilic material comprises one of UV curing or heat curing the hydrophilic material.

## Patentansprüche

1. Ultraschallwandler-Schnittstellenpolster, umfassend:
eine erste Substratschicht (120), die eine erste Oberfläche und eine zweite Oberfläche aufweist;
eine hydrophile Schicht (130), die auf der ersten Oberfläche der ersten Substratschicht gebildet ist, wobei die hydrophile Schicht dazu konfiguriert ist, hydriert zu sein, um eine akustische Kopplung zwischen einem Ultraschallwandler und einem Patienten bereitzustellen;
eine zweite Substratschicht (140), die eine dritte Oberfläche und eine vierte Oberfläche aufweist;
eine erste Haftschicht (125), die auf der dritten Oberfläche der zweiten Substratschicht gebildet ist und dazu konfiguriert ist, an der zweiten Oberfläche der ersten Substratschicht zu haften; und
eine zweite Haftschicht (145), die auf der vierten Oberfläche der zweiten Substratschicht gebildet ist und dazu konfiguriert ist, an einem Betriebsabschnitt eines Ultraschallwandlers zu haften,
**dadurch gekennzeichnet, dass**:
die erste Haftschicht ein nicht-silikonbasiertes Haftmittel, einschließlich eines Acrylkautschuk-basierten Haftmaterials oder eines Synthesekautschuk-basierten Haftmaterials, umfasst, und die zweite Haftschicht ein silikonbasiertes Haftmittel umfasst, und wobei das nicht-silikonbasierte Haftmittel eine erste Abziehkraft zeigt, die stärker als eine zweite Abziehkraft des silikonbasierten Haftmittels ist.

2. Ultraschallwandler-Schnittstellenpolster nach Anspruch 1, wobei mindestens eine der ersten Substratschicht und der zweiten Substratschicht ein Polyurethanfilmmaterial umfasst.

3. Ultraschallwandler-Schnittstellenpolster nach Anspruch 2, wobei das Polyurethanfilmmaterial eine Dicke im Bereich von etwa 0,025 Millimeter (mm) bis 1,00 mm aufweist.

4. Ultraschallwandler-Schnittstellenpolster nach einem der vorhergehenden Ansprüche, wobei die hydrophile Schicht eines der Folgenden umfasst: ein durch ultraviolettes Licht, UV-Licht, härtbares hydrophiles Material oder ein durch Wärme härtbares hydrophiles Material.

5. Ultraschallwandler-Schnittstellenpolster nach Anspruch 1, wobei die zweite Haftschicht eine Dicke im Bereich von etwa 0,025 Millimeter (mm) bis 0,2 mm aufweist.

6. Ultraschallwandler-Schnittstellenpolster nach Anspruch 5, wobei die zweite Haftschicht ein Beschichtungsgewicht im Bereich von 100 Gramm pro Quadratmeter (g/m²) bis 200 g/m² aufweist.

7. Ultraschallwandler-Schnittstellenpolster nach einem der vorhergehenden Ansprüche, ferner umfassend:
eine Trennschicht, die über der zweiten Haftschicht bereitgestellt ist,
wobei die Trennschicht vor dem Haften der zweiten Haftschicht auf dem Ultraschallwandler abzuziehen ist.

8. Verfahren zum Herstellen eines Einweg-Ultraschallwandler-Schnittstellenpolsters, wobei das Verfahren Folgendes umfasst:
Aufbringen eines hydrophilen Materials auf eine erste Oberfläche einer ersten Substratschicht (120), um eine hydrophile Schicht (130) zu bilden,
wobei das hydrophile Material dazu konfiguriert ist, hydriert zu sein, um eine akustische Kopplung zwischen dem Patienten und einem Ultraschallwandler bereitzustellen;
Härten der hydrophilen Schicht;
Aufbringen eines ersten Haftmittels auf eine dritte Oberfläche einer zweiten Substratschicht (140), um eine erste Haftschicht (125) zu bilden,
Aufbringen eines zweiten Haftmittels auf eine vierte Oberfläche der zweiten Substratschicht, um eine zweite Haftschicht (145) zu bilden;
Verbinden der ersten Substratschicht mit der zweiten Substratschicht über die erste Haftschicht; und
Zuschneiden der verbundenen ersten Substratschicht und zweiten Substratschicht auf eine gewünschte Größe und Form, um das Einweg-Ultraschallwandler-Schnittstellenpolster zu bilden,
wobei die erste Haftschicht ein nicht-silikonbasiertes Haftmittel und die zweite Haftschicht ein silikonbasiertes Haftmittel umfasst und wobei das nicht-silikonbasierte Haftmittel eine erste Abziehkraft zeigt, die stärker als eine zweite Abziehkraft des silikonbasierten Haftmittels ist.

9. Verfahren nach Anspruch 8, wobei mindestens eine der ersten Substratschicht oder der zweiten Substratschicht ein Polyurethanmaterial umfasst.

10. Verfahren nach Anspruch 9, wobei das Polyurethanmaterial eine Dicke im Bereich von etwa 0,025 Millimeter (mm) bis 1,0 mm aufweist.

11. Verfahren nach einem der Ansprüche 8-10, ferner umfassend:
Aufbringen einer Trennschicht auf die zweite Haftschicht.

12. Verfahren nach Anspruch 8, wobei die zweite Haftschicht eine Dicke im Bereich von etwa 0,025 Millimeter (mm) bis 0,2 mm aufweist.

13. Verfahren nach Anspruch 12, wobei das Aufbringen einer zweiten Haftschicht Folgendes umfasst:
Beschichten des silikonbasierten Haftmittels mit einem Beschichtungsgewicht im Bereich von 100 Gramm pro Quadratmeter (g/m²) bis 200 g/m².

14. Verfahren nach einem der Ansprüche 8-13, wobei das hydrophile Material eines der Folgenden umfasst: ein durch ultraviolettes Licht, UV-Licht, härtbares hydrophiles Material oder ein durch Wärme härtbares hydrophiles Material und wobei Härten des hydrophilen Materials eines von UV-Härten oder Wärmehärten des hydrophilen Materials umfasst.

## Revendications

1. Tampon d'interface de transducteur ultrasonore, comprenant :
une première couche de substrat (120) ayant une première surface et une deuxième surface ;
une couche hydrophile (130) formée sur la première surface de la première couche de substrat, la couche hydrophile étant configurée pour être hydratée afin de fournir un couplage acoustique entre un transducteur ultrasonore et un patient ;
une deuxième couche de substrat (140) ayant une troisième surface et une quatrième surface ;
une première couche adhésive (125) formée sur la troisième surface de la deuxième couche de substrat et configurée pour adhérer à la deuxième surface de la première couche de substrat ; et
une deuxième couche adhésive (145) formée sur la quatrième surface de la deuxième couche de substrat et configurée pour adhérer à une partie opérationnelle d'un transducteur ultrasonore,
**caractérisé en ce que** :
la première couche adhésive comprend un adhésif sans silicone comprenant un matériau adhésif à base de caoutchouc acrylique ou un matériau adhésif à base de caoutchouc synthétique, et la deuxième couche adhésive comprend un adhésif à base de silicone, et dans lequel l'adhésif sans silicone présente une première force de retrait supérieure à une deuxième force de retrait de l'adhésif à base de silicone.

2. Tampon d'interface de transducteur ultrasonore selon la revendication 1, dans lequel au moins l'une de la première couche de substrat et de la deuxième couche de substrat comprend un matériau de film de polyuréthane.

3. Tampon d'interface de transducteur ultrasonore selon la revendication 2, dans lequel le matériau de film de polyuréthane a une épaisseur allant d'environ 0,025 millimètre (mm) à 1,00 mm.

4. Tampon d'interface de transducteur ultrasonore selon l'une quelconque des revendications précédentes, dans lequel la couche hydrophile comprend l'un des éléments suivants : un matériau hydrophile durcissable à la lumière ultraviolette (UV) ou un matériau hydrophile durcissable à la chaleur.

5. Tampon d'interface de transducteur ultrasonore selon la revendication 1, dans lequel la deuxième couche adhésive a une épaisseur comprise entre environ 0,025 millimètre (mm) et 0,2 mm.

6. Tampon d'interface de transducteur ultrasonore selon la revendication 5, dans lequel la deuxième couche adhésive a un poids de revêtement dans une plage de 100 grammes par mètre carré (gsm) à 200 gsm.

7. Interface de transducteur ultrasonore selon l'une quelconque des revendications précédentes, comprenant en outre :
une couche de libération disposée sur la deuxième couche adhésive,
dans lequel la couche de libération doit être retirée avant de coller la deuxième couche adhésive au transducteur ultrasonore.

8. Procédé de fabrication d'un tampon d'interface de transducteur ultrasonore jetable, le procédé comprenant :
l'application d'un matériau hydrophile sur une première surface d'une première couche de substrat (120) pour former une couche hydrophile (130),
dans lequel le matériau hydrophile est configuré pour être hydraté afin de fournir un couplage acoustique entre le patient et un transducteur ultrasonore ;
le durcissement de la couche hydrophile ;
l'application d'un premier adhésif sur une troisième surface d'une deuxième couche de substrat (140) pour former une première couche adhésive (125),
l'application d'un deuxième adhésif sur une quatrième surface de la deuxième couche de substrat pour former une deuxième couche adhésive (145) ;
la liaison de la première couche de substrat à la deuxième couche de substrat via la première couche adhésive ; et
le coupage de la première couche de substrat collée et de la deuxième couche de substrat à une taille et une forme pour former le tampon d'interface du transducteur ultrasonore jetable,
dans lequel la première couche adhésive comprend un adhésif sans silicone et la deuxième couche adhésive comprend un adhésif à base de silicone, et dans lequel l'adhésif sans silicone présente une première force de retrait supérieure à une deuxième force de retrait de l'adhésif à base de silicone.

9. Procédé selon la revendication 8, dans lequel au moins l'une de la première couche de substrat ou de la deuxième couche de substrat comprend un matériau polyuréthane.

10. Procédé selon la revendication 9, dans lequel le matériau en polyuréthane a une épaisseur comprise entre environ 0,025 millimètre (mm) et 1,00 mm.

11. Procédé selon l'une quelconque des revendications 8 à 10, comprenant en outre :
l'application d'une couche de protection sur la deuxième couche adhésive.

12. Procédé selon la revendication 8, dans lequel la deuxième couche adhésive a une épaisseur comprise entre environ 0,025 millimètre (mm) et 0,2 mm.

13. Procédé selon la revendication 12, dans lequel l'application d'un deuxième adhésif comprend :
le revêtement de l'adhésif à base de silicone avec un poids de revêtement compris entre 100 grammes par mètre carré (gsm) et 200 gsm.

14. Procédé selon l'une quelconque des revendications 8 à 13, dans lequel le matériau hydrophile comprend l'un parmi : un matériau hydrophile durcissable par lumière ultraviolette (UV) ou un matériau hydrophile durcissable à la chaleur, et dans lequel le durcissement du matériau hydrophile comprend l'un parmi le durcissement par UV ou le durcissement à la chaleur du matériau hydrophile.
